# EUROPEAN PATENT APPLICATION

(11) **EP 0 764 911 A1**
(43) Date of publication of application: **26.03.1997**
(21) Application number: 96202340.4
(22) Date of filing: 22.08.1996
(51) Int. Cl.: G06F 17/60

(54) **Medical information communications system and method**

(30) Priority: 22.08.1995 US 518081
(71) Applicant: EMX L.C.C., New York, New York 10022 (US)
(72) Inventor: Loughlin, Donald, Hauppauge, New York 11788 (US); Bernstein, Joseph, New York, New York 10029 (US)
(74) Representative: de Bruijn, Leendert C.

(57) **Abstract**

The present invention is directed to a global emergency medical information communications system and method for delivering medical information via credit card transaction terminals located in emergency rooms, emergency vehicles, and other remote locations around the world. The various embodiments disclosed incorporate a medical information database capable of delivering medical information to transaction terminals. The system and method incorporates the use of existing public switched telephone networks, existing credit transaction networks, or other networks for effecting the delivery of medical information.

In accordance with the invention, a subscriber to the system swipes a subscriber card, registered credit card, or other registered access card at a credit card transaction terminal. The request is then communicated via either the public switched telephone network or existing credit transaction network to the central medical information database. The database then verifies the cards and delivers the associated subscriber medical information back through either network to be printed at the transaction terminal and/or at a designated facsimile machine.

## Description

### FIELD OF THE INVENTION

The present invention is directed to a medical information communications system and method for distributing an individual's personal medical information to emergency rooms, emergency vehicles, and other remote locations around the world using low cost, globally standardized credit card transaction terminals connected to new or existing global information networks.

### BACKGROUND OF THE INVENTION

It is recognized throughout the global medical community that rapid access to an individual's personal medical file and/or medical history is crucial for providing timely and effective treatment to an ill or injured person in an emergency. Individual mobility and global travel make such access extremely difficult using existing technologies. Current and proposed systems to provide medical information rely either on the creation of expensive communications infrastructures to connect with a central database (see for example U.S. Patent No. 4,491,725 to Pritchard and U.S. Patent No. 4,858,121 to Barber et al.) or on special purpose computer systems (smart cards, microchip cards, and the like) to read and update a portable patient record (see for example U.S. Patent No. 5,291,399 to Chaco). Other systems for delivery of emergency medical information rely on live telephone operators responding over the phone or transmitting a fax. However, none of these systems address the difficult problems associated with delivering medical information instantly to emergency rooms, emergency vehicles, or other remote locations throughout the world in a cost effective manner using existing global infrastructures and low cost, universally available, standardized credit card transaction terminals and printers.

There exists today international credit card transaction networks which allow for the instantaneous delivery throughout the world of credit approval authorization codes to any location which has a credit card transaction terminal. However, existing international credit transaction networks only deliver credit approval authorization codes and do not provide for the delivery of medical or other information.

Accordingly, it is an object of the present invention to provide a system and method for delivering information around the world using credit card transaction terminals.

It is another object of the present invention to provide a system and method for delivering information around the world using existing credit card transaction networks.

It is another object of the present invention to provide a system and method for delivering medical information around the world to emergency rooms, emergency vehicles, and other remote locations.

It is another object of the present invention to provide a system and method for delivering medical information around the world to credit card transaction terminals using customized or existing credit cards, magnetic strip cards, integrated circuit cards, bar code cards or other forms of access cards (collectively, "access cards").

It is another object of the present invention to provide a system and method for delivering medical information and image files to any designated fax machine using credit card transaction terminals.

It is another object of the present invention to provide a system and method for delivering medical information and image files using existing unmodified credit card transaction terminals.

### SUMMARY OF THE INVENTION

The present invention (known currently as "MEDBANK") meets the above and other objects by providing a centralized medical information system that can deliver emergency medical information and image files to credit card transaction terminals and fax machines located in emergency rooms, emergency vehicles and other remote locations to persons who carry registered access cards. The present invention is implementable using new or existing information networks and/or new or existing transaction terminals, thereby allowing for simple, flexible, cost effective, worldwide implementation and distribution of medical information.

Preferably, persons interested in having the ability to have their medical information delivered to remote locations subscribe to a service utilizing a medical information system employing the present invention. The subscriber's medical information and image files are stored in a central medical information database accessible from a central medical information system. The medical information can include emergency contact names and telephone numbers, physician names and telephone numbers, drug reactions, allergies, medications, and additional relevant information, including image files of EKGs, MRIs, x-ray films, and other medical image records. Each subscriber is also issued an access card (a "MEDBANK card"), a card number, and a personal I.D. number (PIN) for accessing the medical information database through the central system. Alternatively or additionally, a subscriber may register his or her existing credit cards, health service cards, drivers license, or other access cards with the medical information system. Thereafter, the person can retrieve medical information in a remote location by using the issued access card or a registered access card.

In a first embodiment, the person swipes his or her MEDBANK card in a custom card transaction terminal (a "MEDBANK terminal") located in an emergency room, emergency vehicle or other remote location. In this embodiment, the MEDBANK terminal is a dedicated medical history terminal which does not process credit card transactions. The MEDBANK terminal dials the central medical information system, identifies the card holder, and requests the medical information stored in the database to be delivered to the terminal. The central database then verifies the subscription card and, upon entry of the person's PIN, delivers the person's medical information to the desired location to be printed on an attached or associated terminal printer. The printout format is similar to a credit card receipt only longer to allow for the person's complete medical record to be printed. Alternatively or in addition, the information, along with image files, can be delivered to a designated fax machine.

In a second embodiment, the person uses an existing credit card, or other access card, that has been registered with the central medical information system and swipes same in the MEDBANK terminal. The MEDBANK terminal then dials the central system, identifies the card number, and requests the medical information to be delivered to the MEDBANK terminal. The central database then compares the card number against an index table to determine if the number corresponds to a subscriber's registered card number. If there is a match, the central database delivers the applicable medical information to the desired location, *e.g*., the terminal printer or designated fax machine.

In a third embodiment, the person swipes his or her MEDBANK card at a networked credit card transaction terminal which is preprogrammed to accept MEDBANK cards. In this embodiment, instead of dialing a merchant bank as the credit card transaction terminal would normally do to process a credit card transaction, the transaction terminal dials the MEDBANK medical information system directly, *i.e*.,it "split dials." The database then verifies the card number and delivers the applicable medical information directly to the existing transaction terminal printer and/or to a designated facsimile machine.

Alternatively, in a fourth embodiment, instead of split dialing as above, the MEDBANK central database system becomes a "member bank" within the credit transaction network. In this embodiment, the credit card transaction terminal calls the merchant bank computer system associated with the terminal and presents the card in the same fashion as the transaction terminal processes credit card transactions. Thereafter, the merchant bank system forwards the information request to a central credit system, for example a MasterCard®, VISA®, or American Express®, central credit computer system, also in the same fashion as a normal credit card transaction. The central credit computer system then forwards the request to the MEDBANK central database system, again in the same fashion as a central credit computer system would forward a credit approval request to a customer bank computer system. The database then verifies the card and forwards the requested medical information back through the existing credit card network to print at the initiating transaction terminal. The requested medical information can also be delivered directly from the database (without going back through the credit card network) by dialing back to the terminal or to a designated facsimile machine via the public switched telephone network (PSTN).

In a fifth embodiment, a person swipes a MEDBANK card or an existing registered credit card at an existing credit card transaction terminal preprogrammed to accept requests for medical information. In this embodiment, a program function key on the transaction terminal is pressed to indicate to the transaction terminal that the following request is not a credit card transaction but a request for medical information. The transaction terminal can either split dial and call the medical database system directly, as described above, or indicate a request to the credit card network system to route the following MEDBANK card number or credit card number to the central medical database system, as described above. The medical database system then verifies the card and forwards the requested medical information back through the existing credit network to print at the initiating transaction terminal or a designated facsimile machine. As with other embodiments, the requested medical information can also be delivered from the central database directly to the terminal or a designated facsimile machine via the public switched telephone network.

In a sixth embodiment, existing transaction terminals are registered with the central medical information system. Specifically, the transaction terminal I.D. number and the telephone number corresponding to the nearest fax machine to the transaction terminal are registered with the medical information system. When a person swipes a MEDBANK card at a registered transaction terminal, the terminal dials the corresponding merchant bank which forwards the request to the central credit computer system which in turn forwards the request to the medical information system. The medical information system then checks the transaction identification number with an index of same to determine the corresponding designated facsimile machine associated with the transaction terminal. The medical information system then dials the facsimile machine and delivers the corresponding medical information to same.

### BRIEF DESCRIPTION OF THE DRAWINGS

Figure 1 is a block diagram illustration of two embodiments of the present invention wherein a MEDBANK terminal is used to process requests for medical information using a MEDBANK card.

Figure 2 is a block diagram illustration of two other embodiments of the present invention wherein a network credit card transaction terminal is preprogrammed to accept a MEDBANK card for the receipt of medical information.

Figure 3 is a block diagram illustration of another embodiment of the present invention wherein a preprogrammed network credit card transaction terminal includes a medical information request function key to direct requests for medical information to the central medical information system.

Figure 4 is a block diagram illustration of another embodiment of the present invention wherein an existing transaction terminal is used to request medical information to be received by a designated fax machine.

Figure 5 is a flow chart showing the operation of a central medical information system.

Figure 6 is a flow chart showing the operation of a MEDBANK transaction terminal.

Figure 7 is a flow chart showing the operation of a preprogrammed transaction terminal.

Figure 8 is a flow chart showing the operation of a preprogrammed transaction terminal wherein the terminal includes a medical request key.

Figure 9 is an illustration of a transaction terminal printout of medical information.

### DESCRIPTION OF THE PREFERRED EMBODIMENTS

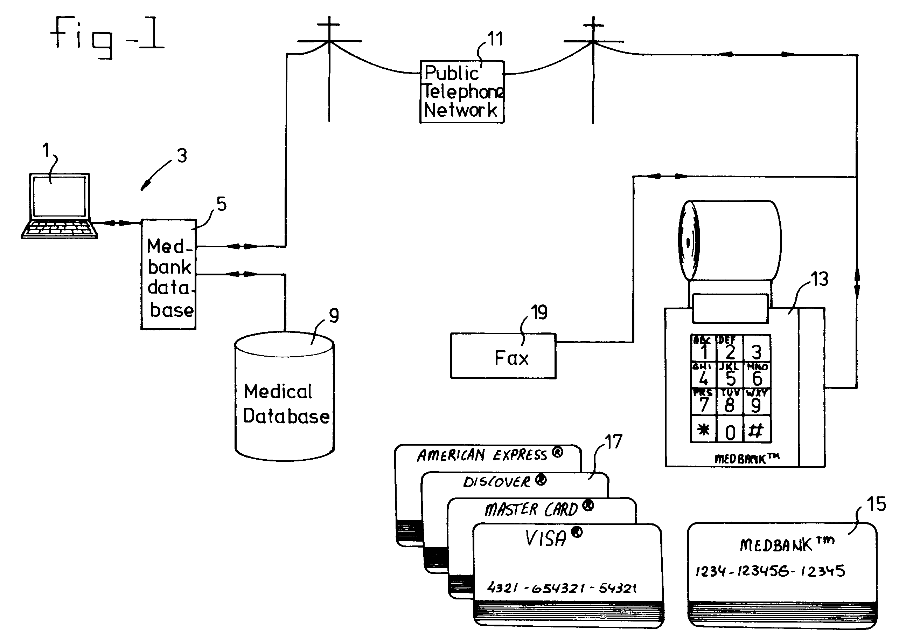

As illustrated in FIGS. 1-4, common to the various embodiments of the present invention is the use of a central medical information system 3 or "host" comprising a computer system 5 having various terminals 1 and a relational medical information database 9. As will become readily apparent to those skilled in the art, the central medical information system can be a mainframe computer system, a minicomputer system, a personal computer system, or, preferably, a network of various computer systems.

To maintain cost efficiencies, the currently preferred hardware is at least two Acer/Altos P7000 multiprocessor computers, linked by private leased lines. The currently preferred operating system is SCO Open Server Enterprise UNIX, which is designed to meet the C2 class of "trust" as defined by the Trusted Computer System Evaluation Criteria. As will become readily apparent to those skilled in the art, the hardware and operating system are not unique and numerous systems can be employed to construct a medical information system in accordance with the present invention.

In a preferred embodiment, the system, including its relational medical information database, are built on a commercial database engine, for example Sculptor, and the programs to access and maintain the database are written in Sculptor 4th Generation Language and in C programming language. The currently preferred database engine and programming languages are not exclusive and the system could be built on a multitude of systems. Preferably, the system is implemented in an asynchronous symmetrically replicated database configuration using N (any number 2 or more) system network servers. Further, the servers are located at geographically diverse locations to reduce the exposure to failure and are interconnected using high speed digital communication circuits.

The database 9 contains related records for all medical information including medical image files associated with each MEDBANK subscriber. Multiple indices are maintained for each record allowing for rapid assembly of a complete summary medical history and a longitudinal history or "snap shot" for any given chronological time. The longitudinal history allows for a time ordered collection of events related to the medical record to be maintained. For example, a longitudinal history of blood pressure would include an entry for each time a subscriber's blood pressure was recorded, in chronological order. A new blood pressure reading would not replace the last reading, but rather create an additional record, placed after the previous record. This allows the reconstruction of the status at any point in time, and provides a complete audit trail of all entries.

Data is entered at any terminal 1 on any node of the central system network and is then asynchronously replicated to all other nodes by use of linked logging files. Each entry in the database simultaneously creates a log entry on the node. The log entries are then replicated to all other nodes, where they are joined to the database at that node. Should a node become unavailable for any reason, the log entries accumulate until the node becomes available again.

To maintain integrity of the data, modifications of database entries are not permitted. Changes in information are implemented by entry of an additional record which is linked to the longitudinal chain for that MEDBANK subscriber. For example, a change of address results in a new address record, which is flagged as the current record. The previous address is not changed and is available in perpetuity.

The present invention is directed to delivering the medical information stored in the medical information database to credit card transaction terminals located in emergency rooms, emergency vehicles, and other remote locations around the world. It is also directed to delivering medical information and image files to designated facsimile machines. Further, as will become readily apparent to those skilled in the art, the information once delivered to a transaction terminal can also be captured and used in other information systems, for example, within a central hospital patient record information system.

FIG. 1 is a block diagram illustrating two embodiments of the delivery system of the present invention. The first embodiment uses a MEDBANK subscriber card 15. The second embodiment uses any currently available credit card or other access card, for example an American Express®, Discover®, MasterCard®, or VISA® credit card 17, which is previously registered with the central medical information system. As shown in FIG. 1, the central medical information system 3 is connected to the public switched telephone network II in a known manner. Also connected to the telephone network 11 is a series of custom MEDBANK terminals 13. The MEDBANK terminal is preferably built using a Verifone Tranz 460 transaction terminal programmed to operate as a MEDBANK terminal. Any other currently available commercial credit card transaction terminals can be used provided the terminal has preferably 32k bytes of programmable memory, a modem, a cardswipe, a keyboard, and an annexed or associated printer. Also shown is an optional fax machine 19, which can also be used for the delivery of medical information and, more particularly, medical image files.

In the first embodiment, a MEDBANK subscriber card 15 is used. Preferably, the MEDBANK card is identical in size and layout to any known commercial credit card, for example a VISA^{·} credit card. Further the location and size of the magnetic stripe is standardized and the format of the data on the stripe is in accordance with the American Banking Association Track II standard as listed in the following table:

**TABLE 1**

| | |
|---|---|
| Start Sentinel | 1 Character (02H) |
| Account Number | Up to 19 Numeric Characters (0 through 9) |
| Field Separator | 1 Character (1 CH) |
| Expiration Date | 4 Characters (MMYY) |
| Discretionary Data | Total of account numbers, expiration date and discretionary data not to exceed 36 characters (0 through 9) |
| End Sentinel | 1 Character (03H) |
| Redundancy Check | 1 Character (LRC) |

Preferably, the first 4 to 6 digits indicate the system and issuer, the next group of digits are the actual membership identification number *i.e*. a subscriber access number, the last digit is a check digit derived from the LUNN base ten algorithm.

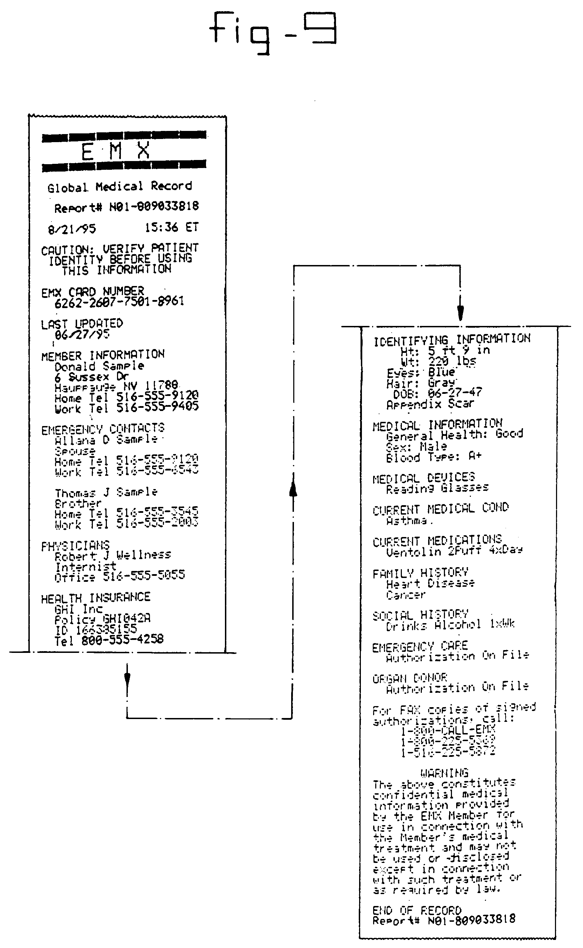

In a first embodiment, a subscriber to the MEDBANK service, or other person, swipes the MEDBANK card in the MEDBANK transaction terminal 13 located in an emergency room, emergency vehicle or other remote location. As with many embodiments, a person has the option of using the terminal keyboard to transmit the information associated with the access card. In this embodiment, the MEDBANK terminal modem dials and establishes a connection with the medical information system, identifies the card holder, and requests the medical information stored in the database be delivered to the MEDBANK terminal. The central database then verifies the subscription card and, upon entry of the person's PIN, delivers the person's medical information to the transaction terminal to be printed. A sample of a transaction terminal printout is shown in FIG. 9. Alternatively, the transaction terminal operator can direct medical information to be delivered to a designated fax machine 19. The central medical information system 3 then separately dials the designated or other selected fax machine and delivers the medical information and/or image files in a known manner using existing wire, cellular, radio, or other telephone systems.

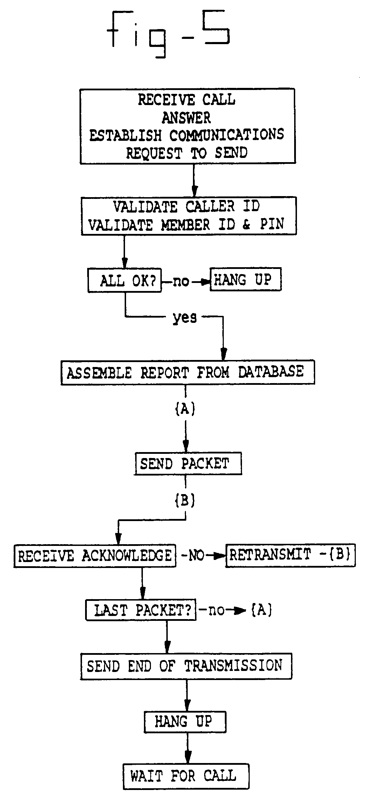

FIG. 5 is a high level flow chart of the program used to operate the central medical information system of various embodiments. The system begins by receiving an incoming call. The central system and terminal modems exchange handshaking signals using the appropriate CCITT or Bell standards to synchronize their transmission speeds and protocols and establish a communications link between the terminal and host. The host then sends a "Request To Send" character. The terminal responds with a request packet consisting of the identification data of the terminal and member and security codes(s). The host examines the request packet, first for data integrity by calculating a checksum on the packet and comparing it to the checksum sent by the terminal. The host then validates the terminal identification and security information with the host database of such data. Finally, the host examines the member identification and security information for its validity. Should an error occur in transmission, several attempts are made to retransmit the data. If either a communication error persists or any of the security criteria is violated, the host terminates the session and reports that an unsuccessful attempt to gain access was attempted.

Upon acceptance of the request packet, the host assembles the data for the subscriber requested from its database(es), formats it for the designated terminal printer and sends the data in the form of data packets to the terminal, one at a time. Each data packet contains a checksum which is examined by the terminal to insure uncorrupted data transmission. If the checksum is valid, the terminal transmits an acknowledgement, else it transmits a negative acknowledgement. The host will retransmit the data packet upon receipt of a negative acknowledgement.

After the terminal acknowledges the transmission of the last data packet, the host sends an "End Of Transmission" character and terminates the session.

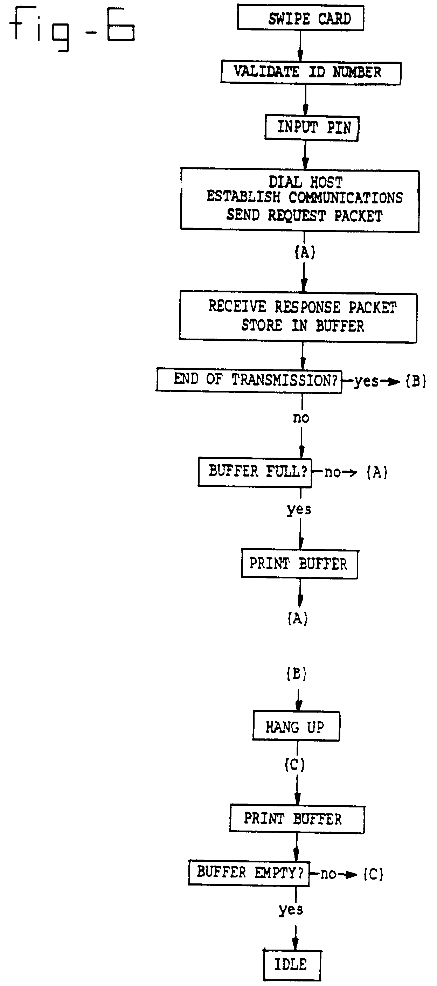

FIG. 6 is a high level flow chart of the program used to operate the MEDBANK transaction terminal. Operation of the terminal begins by swiping a card through the card swipe slot. The terminal then performs basic validation of the member identification number in accordance with established criteria for numeric range and structure. The terminal user then enters the subscriber's Personal Identification Number (PIN) for security. The terminal then dials or otherwise initiates connection to the host. The terminal and host modems exchange handshaking signals using the appropriate CCITT or Bell standards to synchronize their transmission speeds and protocol and establish a communications link between the terminal and host. The host then sends a "Request To Send" character.

The terminal constructs a data packet containing identification information for itself and the member, and security data including an encrypted PIN. The terminal then transmits this data packet to the host. The host responds either with an acknowledgement or negative acknowledgement. Upon receipt of a negative acknowledgement the terminal retransmits the data packet.

The host then sends data packets containing the medical history to be printed at the terminal. Each data packet contains a checksum which the terminal examines to insure uncorrupted data has been received. If the checksum is not valid, the terminal transmits a negative acknowledgement The host retransmits the data packet upon receipt of a negative acknowledgement.

Upon acceptance of the data packet, the terminal calculates if it has sufficient space to store it in its printer buffer. If there is space, the terminal stores the data packet and sends an acknowledgement to the host. If there is not sufficient space in the print buffer for the data packet, the terminal prints data until enough space is created to store the new data packet. The terminal then stores the packet and sends an acknowledgement.

When the terminal receives an "End Of Transmission" character from the host, it terminates the communication path and prints or completes printing the received data.

In a alternate embodiment, a registered credit card 17 or similar access card is swiped through the MEDBANK terminal 13. The MEDBANK terminal then dials the central medical information system 3, identifies the card number, and requests the medical information be delivered to the MEDBANK terminal. The central system 3 then compares the card number against an index table to determine if the number corresponds to a subscriber's registered credit card number. If there is a match, the central system delivers the applicable medical information to the desired location, either to the transaction terminal or a to a fax machine as described above.

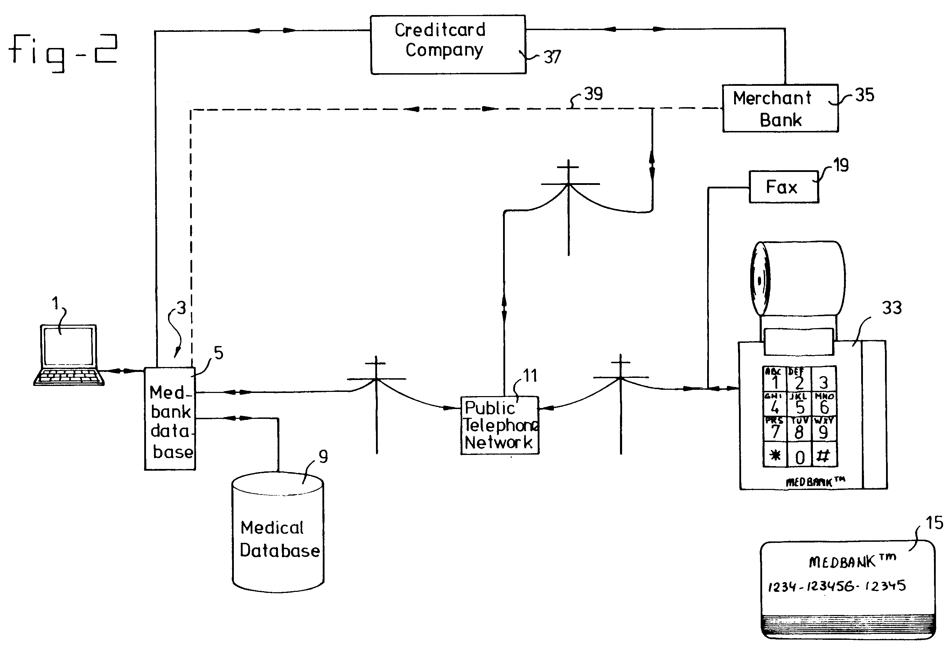

FIG. 2 is a block diagram of two other embodiments of the present invention. In one embodiment, the person swipes a MEDBANK card at a network credit card transaction terminal 33 which is preprogrammed to accept MEDBANK, registered credit cards, or similar access cards and to process normal credit transactions. In this embodiment, instead of dialing a merchant bank as the credit card transaction terminal would normally do to process a credit card transaction, the transaction terminal dials the MEDBANK medical information system directly over the public switched telephone network 11, *i.e*., it "split dials." The database then verifies the card number and delivers the applicable medical information directly to the existing transaction terminal printer and/or to a designated facsimile machine 19.

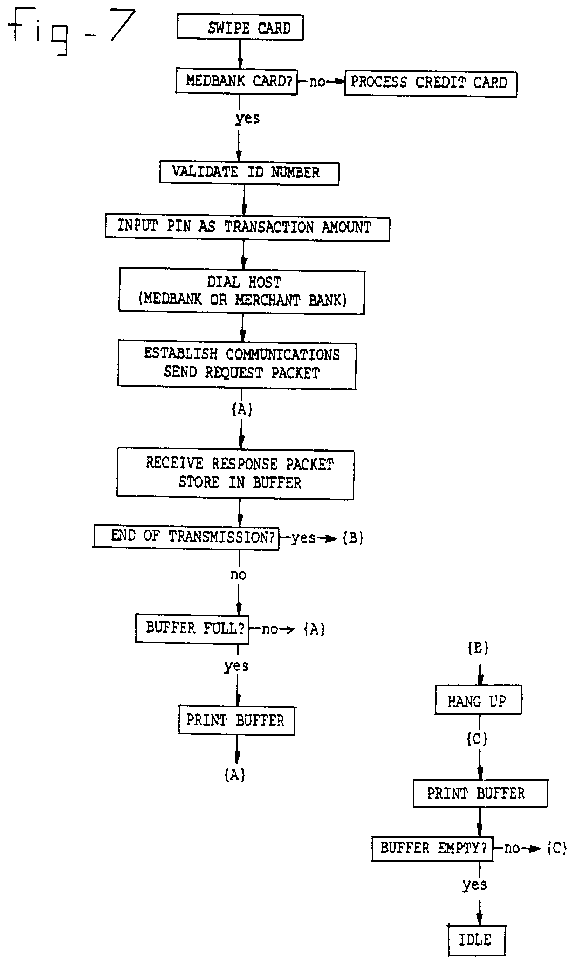

FIG. 7 is a high level flow chart of the program used to operate the transaction terminal 33. Operation of the terminal begins by swiping a card through the card swipe slot. The terminal performs basic validation of the member identification number in accordance with established criteria for numeric range and structure. If the account number read from the card is within the range defined for credit cards, the terminal processes a standard credit card transaction, employing one of the well known protocols in use.

Where the account number read from the card falls within the range defined for medical history identification cards the terminal user is then prompted to enter a Personal Identification Number (PIN) for security. The terminal then dials or otherwise Imitates connection to the host directly or to an intermediate merchant bank or processor. Where connection is through an intermediary, all data packets are received and forwarded through the existing credit card processing infrastructure. Both the terminal and host communicate through the intermediary as though they were directly connected.

The terminal and host modems exchange handshaking signals using the appropriate CCITT or Bell standards to synchronize their transmission speeds and protocols and establish a communications link between the terminal and host. The host then sends a "Request To Send" character.

The terminal constructs a data packet containing identification information for itself and the member, and security data including an encrypted PIN. The terminal then transmits this data packet to the host. The host responds either with an acknowledgement or negative acknowledgement. Upon receipt of a negative acknowledgement the terminal retransmits the data packet.

The host sends data packets containing the medical history to be printed at the terminal. Each data packet contains a checksum which the terminal examines to insure uncorrupted data has been received. If the checksum is not valid, the terminal transmits a negative acknowledgement. The host retransmits the data packet upon receipt of a negative acknowledgement.

Upon acceptance of the data packet, the terminal calculates if it has sufficient space to store it in its printer buffer. If there is space, the terminal stores the data packet and sends an acknowledgement to the host. If there is not sufficient space in the print buffer for the data packet, the terminal prints data until enough space is created to store the new data packet. The terminal then stores the packet and sends an acknowledgement.

When the terminal receives an "End Of Transmission" character from the host, it terminates the communications path and prints or completes printing of the received data.

Alternatively, in another embodiment, instead of split dialing, the request for medical information is sent through a credit transaction network to the central medical information system 3, the medical information system 3 being registered as a customer bank member of the credit transaction network. In this embodiment, the credit card transaction terminal 33 is programmed to call the merchant bank computer system 35 associated with the terminal and present the card information in the same fashion as the transaction terminal 33 normally processes credit card transactions. Thereafter, the merchant bank or credit clearing house 35 forwards the information request to a central credit transaction system 37, for example a MasterCard®, VISA®, or American Express® central credit transaction computer system, also in the same fashion as a normal credit card transaction. The central credit computer system 37 then forwards the request to the medical information system 3, again in the same fashion as a central credit computer system would forward a credit approval request to a customer bank computer system. The database then verifies the card and forwards the requested medical information back through the existing credit transaction network to print at the initiating transaction terminal, which has been preprogrammed to print all of the downloaded medical information.

Further, when a transaction terminal is connected to a credit clearing house 35, the information request can alternatively be routed directly to the medical information system 3 by way of a direct line 39 thereby bypassing the credit card system 37.

Currently, the existing credit card transaction network has limited print back ability, *i.e*., only approximately 25 bytes of information can be printed from a customer bank back to a transaction terminal without reprogramming the terminal. This information typically includes a credit authorization code and other information. In this alternate embodiment, the existing credit card transaction network is modified and/or reprogrammed to allow a complete medical history to he printed at the transaction terminal. Alternatively, the requested medical information can also be delivered directly from the medical information system 3 to the terminal or a designated facsimile machine via the public switched telephone network 11 (without going back through the credit card network) by allowing the system 3 to dial back to the terminal 33 via the public telephone network 11.

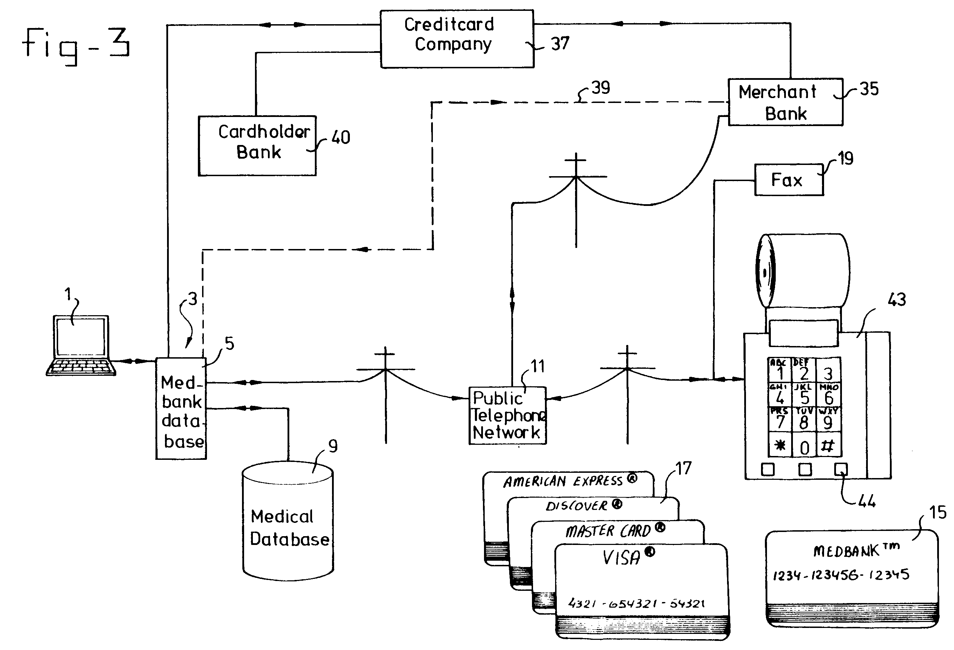

In yet another embodiment, as illustrated in FIG. 3, a person swipes a MEDBANK card 15 or an existing registered credit card 17 at an existing credit card transaction terminal 43 preprogrammed to accept requests for medical information. In this embodiment, a program function key 44 on the transaction terminal is pressed to indicate to the transaction terminal that the following request is not a credit card transaction but a request for medical information. The transaction terminal can either split dial and call the medical database system directly, as described above, or indicate a request to the credit transaction network system to route the following credit card number to the medical database system, as described above. The medical database system 3 then verifies the card and forwards the requested medical information back through the existing credit network to print at the initiating transaction terminal or a designated facsimile machine. As with other embodiments, the requested medical information can also be delivered directly to the terminal or a designated facsimile machine via the public switched telephone network 11. When the medical request key 44 is not pressed the transaction action terminal processes credit transactions through the network to a card holder bank 40.

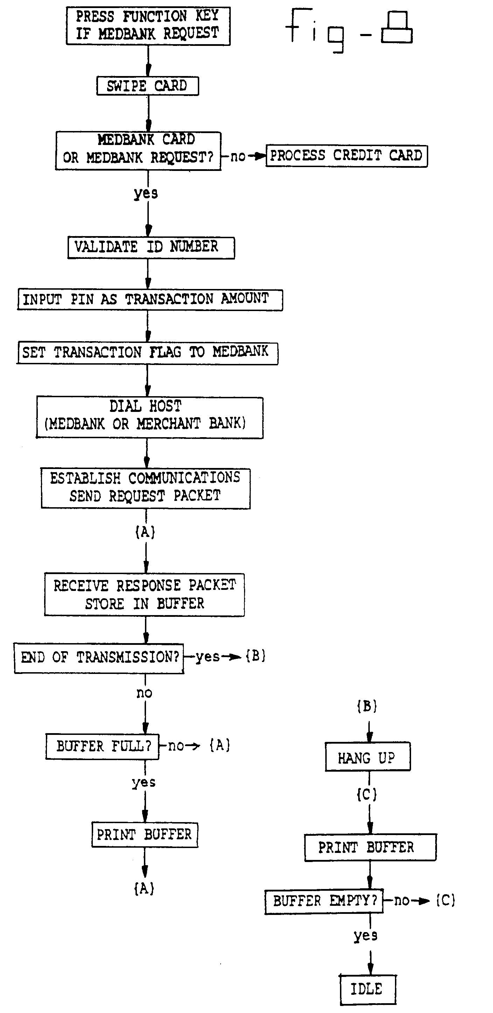

FIG. 8 is a high level block diagram of the operation of a transaction terminal 43. Operation of the terminal begins by swiping a card through the card swipe slot, or by pressing a keyboard key(s) to indicate that the following card swipe is a medical history request. The terminal performs basic validation of the member identification number in accordance with established criteria for numeric range and structure. If the account number read from the card is within the range defined for credit cards, and the keyboard request for medical history was not pressed, the terminal processes a standard credit card transaction, using one of the well known protocols in use.

Where the account number read from the card falls within the range defined for medical history identification cards, or a keyboard request for medical history was made, the terminal user is then prompted to enter a Personal Identification Number (PIN) for security. The terminal then dials or otherwise initiates connection to the host directly or to an intermediate merchant bank or processor. Where connection is through an intermediary, all data packets are received and forwarded through the existing credit transaction network infrastructure. Both the terminal and host communicate through the intermediary as though they were directly connected.

The terminal and host modems exchange handshaking signals using the appropriate CCITT or Bell standards to synchronize their transmission speeds and protocols and establish a communications link between the terminal and host. The host then sends a "Request To Send" character.

The terminal constructs a data packet containing identification information for itself and the member, and security data including an encrypted PIN and a flag (single character) to identify the transaction as a request for medical records. This flag is the same one used to differentiate between manually entered credit card numbers, and swiped credit transactions. In this application, it will be set to a unique state to designate a medical history request.

The terminal then transmits this data packet to the host. The host responds either with an acknowledgement or a negative acknowledgement. Upon receipt of a negative acknowledgement the terminal retransmits the data packet.

The host sends data packets containing the medical history to be printed at the terminal. Each data packet contains a checksum which the terminal examine to insure uncorrupted data has been received. If the checksum is not valid, the terminal transmits a negative acknowledgement. The host retransmits the data packet upon receipt of a negative acknowledgement.

Upon acceptance of the data packet, the terminal calculates if it has sufficient space to store it in its printer buffer. If there is space, the terminal stores the data packet and send an acknowledgement to the host. If there is not sufficient space in the print buffer for the data packet, the terminal prints data until enough space is created to store the new data packet. The terminal then stores the packet and sends an acknowledgement.

When the terminal receives an "End Of Transmission" character from the host, it terminates the communications path and prints or completes printing of the received data.

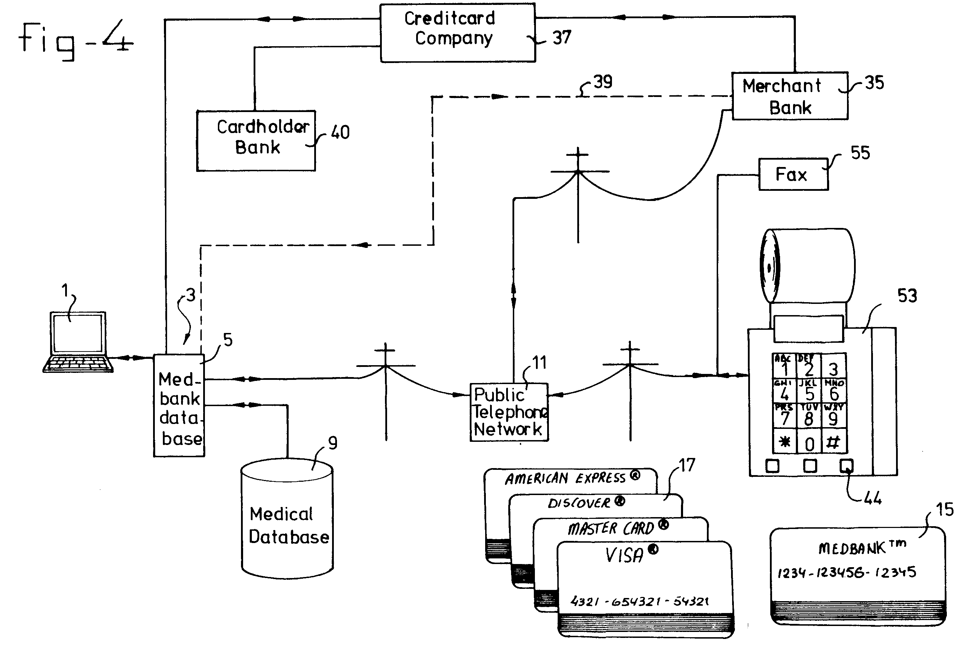

In yet another embodiment, to avoid the need to reprogram an existing transaction terminal, a system of registered transaction terminals can be used. In this embodiment, as illustrated in FIG. 4, anyone having an existing transaction terminal 53 can register the terminal identification number ("TID") and the telephone number corresponding to the nearest fax machine 55 with the medical information system 3. When a MEDBANK card 15, a registered credit card 17, or other registered access card is swiped at the transaction terminal, the request is sent to the merchant bank or credit clearing house 35, then to the central credit system 37, and then to the medical information system 3. The medical information system then checks the TID against a table of TID's to determine first whether the particular transaction terminal is registered and, if so, what the registered designated fax machine telephone number is. Thereafter, the medical information system 3 dials the fax machine 55 and delivers the medical information to same.

Once an embodiment of a medical information system according to the present invention is in place, a typical request for medical information would be as follows (all times approximate, are for illustration only, and can vary with embodiments and with installed system specifications).

| TIME (min:sec) | EVENT |
|---|---|
| 00:00 | A MEDBANK subscriber is stricken by chest pains in a restaurant. |
| 00:30 | The restaurant manager summons Emergency Medical Services by calling 911. |
| 01:00 | The subscriber gives the manager his MEDBANK card and access code. |
| 01:30 | The manager swipes the card through the restaurant's credit card terminal. |
| 01:31 | The terminal recognizes the card as a MEDBANK card by the account number sequence encoded on the magnetic stripe. |
| 01:33 | The terminal then dials the computer access port assigned to it and transmits a request to the credit card transaction network. |
| 01:45 | The credit card transaction network routes the request through its digital data network to MEDBANK. |
| 01:50 | MEDBANK'S computer receives the request, retrieves the member data from the database, verifies the access code and formats the response, containing the subscriber's emergency medical history. |
| 01:55 | The MEDBANK computer sends the data to the credit card transaction network addressed to the restaurant's credit card terminal. |
| 02:00 | The terminal starts printing the subscriber's emergency medical history on its receipt printer. |
| 02:45 | Printing completed. |
| 03:00 | EMS arrives and is able to immediately start treatment, with the knowledge that they are aware of the subscriber's medical history, drug allergies and the like. |

In the above example, if a credit card transaction terminal was not available in the restaurant, the subscriber's card could have been swiped at a wireless MEDBANK terminal located in the ambulance, at a MEDBANK terminal located in an Emergency Room, or at any other existing transaction terminal.

Further, as will become readily apparent to those skilled in the art, the present invention can be used to deliver other information besides medical information through existing or new credit transaction networks. For example, a subscriber could swipe an access card at a transaction terminal and have sports data, stock quotes, lottery numbers, personal messages, e-mail messages, and the like printed at the transaction terminal and/or at a designated fax machine.

Although the invention has been described in connection with various preferred embodiments, those skilled in the art will appreciate that numerous modifications and adaptations may be made without departing from the spirit and scope of the invention as set forth in the following claims.

## Claims

1. A method for delivering medical information from a central information system to a terminal, **characterized** by using a transaction terminal, activating the transaction terminal, establishing a connection between the transaction terminal and the central information system and delivering medical information from the central information system over the established connection to the transaction terminal.

2. A method according to claim 1, wherein medical information is delivered from the central system to a facsimile machine assigned to the transaction terminal.

3. A method according to claim 2, wherein the medical information includes image files.

4. A method according to one of the claims 1-3, wherein the activating step includes the step of swiping an access card at the transaction terminal.

5. A method according to claim 4, wherein the access card is a credit card preregistered with the central information system.

6. A method according to claim 4, wherein the access card is a subscriber card preregistered with the central information system.

7. A method according to claim 1, wherein the establishing a connection step includes the step of using a modem in the transaction terminal to connect to a modem at the central information system.

8. A method according to claim 7, wherein the terminal modem to central information system modem connection is made over a public switched telephone network.

9. A method according to claim 7, wherein the transaction terminal can also process credit transactions.

10. A method according to claim 1, wherein the central information system is a member of a credit transaction network and wherein the establishing a connection step includes the steps of:
i) connecting to the credit transaction network;
ii) sending an information request over the network; and
iii) receiving and accepting the information request at the central information system.

11. A method according to claim 10, wherein the transaction terminal can be used to process both requests for medical information and credit transactions.

12. A method according to claim 10, wherein the connecting to the credit transaction network step includes the step of activating a key on the transaction terminal.

13. A method according to claim 10, wherein the transaction terminal having an identification number registered with the central information system, an associated facsimile telephone number, and a facsimile machine, comprising the steps of:
a) activating the transaction terminal;
b) establishing a connection to the credit transaction network;
c) transmitting the transaction terminal identification number over the credit transaction network to the central information system;
d) determining the facsimile telephone number assigned to the transaction terminal identification number;
e) using the assigned facsimile number to connect to the facsimile machine over a public switched telephone network; and
f) delivering medical information to the facsimile machine.

14. A system for delivering medical information comprising;
subscribers;
a central medical information database for storing and retrieving subscriber medical information for the subscribers to the system,**characterized** by a transaction terminal having connection means to connect to the central medical information database; transmit means to transmit a subscriber access number to the central medical information database and receiving means to receive and print the subscriber medical information indexed to the transmitted subscriber access number.

15. A system according to claim 14, wherein the connection means uses an existing public telephone network to connect the transaction terminal directly to the central medical information database.

16. A system according to claim 15, wherein the connection means is interfaced to a credit transaction network to transmit the subscriber access number over the network to the central medical information database and the receiving means is interfaced to the network to receive and print the medical information at the transaction terminal.

17. A device for retrieving medical information comprising a card having an access code associated with a medical information database and a magnetic strip for storing and retrieving the access code at a transaction terminal.

18. A device according to claim 17, wherein the card is a credit card.

19. A device according to claim 17, where the card is a driver's license.

20. A transaction terminal for retrieving medical information comprising:
a keyboard;
a program memory;
a magnetic card swipe;
a modem;
an associated printer;
a processor to control the keyboard, card swipe, modem, and printer in response to a program stored in the memory, the program designed to control the processor to activate the modem to connect to a medical information database, to request medical information corresponding to an access code retrieved from the cardswipe or keyboard, and to print the requested information on the printer.

21. A transaction terminal according to claim 20, wherein the program is also designed to use the keyboard, printer, modem, and cardswipe to process credit transactions.

22. A system for delivering medical information comprising:
a medical information database;
a first network interface to connect the database to a network;
a transaction terminal having an associated printer, a facsimile, a second network interface to connect the terminal to the network, and means to request medical information from the database to be delivered over the network to the transaction terminal printer.

23. A system according to claim 22, wherein the network is a public switched telephone network.

24. A system according to claim 22, wherein the network is a credit transaction network.
